# EUROPEAN PATENT SPECIFICATION

(11) **EP 0 244 860 B1**
(45) Date of publication and mention of the grant of the patent: **07.04.1993**
(21) Application number: 87106618.9
(22) Date of filing: 07.05.1987
(51) Int. Cl.: C07H 21/04

(54) **Polynucleotide probes and a method for their preparation**
Polynucleotide Probe und Verfahren zu ihrer Herstellung
Sondes polynucléotidiques et leur méthode de préparation

(30) Priority: 07.05.1986 US 860717
(43) Date of publication of application: 11.11.1987
(73) Proprietor: ENZO BIOCHEM, INC., New York, N.Y. 10013 (US)
(72) Inventor: Mao, David T., Harrison, NJ 07029 (US); Cook, Alan F., Cedar Grove, NJ 07009 (US)
(74) Representative: VOSSIUS & PARTNER

(56) References cited:
- EP-A- 0 151 001
- EP-A- 0 209 996
- WO-A-86/02929
- CHEMICAL ABSTRACTS, vol. 95, 1981, Columbus, OH (US); L.H.SCHULMAN et al., p. 310, no. 57576h#
- CHEMICAL ABSTRACTS, vol. 106, 1987, Columbus, OH (US); A.REISFELD et al., p. 291, no. 115888v#
- NUCLEIC ACIDS RESEARCH, vol. 9, 1981; pp. 1203-1217#

## Description

The present invention relates to a method for the preparation of polynucleotide probes. The polynucleotide probes comprise a polynucleotide moiety, a hydrazide compound, and a reporting moiety. The reporting moiety is attached to the hydrazide compound which in turn is attached to the 4-position of a deaminated cytosine base.

A number of diagnostic methods are available for the detection of pathogens or infectious agents. The traditional method comprises cultivating the pathogen until it could be identified, for example, by morphology and/or color. This method has drawbacks in that not all pathogens can be cultivated, and in that the method is time consuming, laborious, relatively non-specific, and relatively insensitive.

This was followed by the immunoassay method, where pathogens are identified by the formation of complexes comprising antibodies with pathogen-specific antigens. Initially, this method comprised the use of radioactive reporter molecules. This method thus has drawbacks in that: radioactivity is required which necessitates the use of special facilities and highly trained workers; aged samples are unstable; there is the problem of cross-reactivity between antigens; antibodies are sensitive to reaction conditions including temperatures, pH, and ionic strength; and target tissue can't be fixed to a support.

Improved immunoassay methods, eg., ELISA, permitted the use of fluorescent or enzymatic reporting moieties. Although radioactivity was no longer a problem, the other drawbacks remained.

A most promising technique for identifying pathogens is that of nucleic acid hybridization. Besides being specific, sensitive, rapid, simple, and versatile, the method allows the identification of pathogens in aged or in highly contaminated samples. This method is also not limited to the detection of pathogens. It can be used to detect the presence of any target polynucleotide, for example, an oncogene.

Early hybridization assays utilized a radioactive isotope in the polynucleotide probe for verification of hybridization. These assays had the drawbacks associated with the use of radioactivity. These drawbacks were obviated by incorporating a fluorescer, chromogen, or enzyme as a reporting moiety in the polynucleotide probe.

The preparation of the latter polynucleotide probes has generally required enzymatic reactions to incorporate the nucleotide comprising the reporting moiety into a particular base sequence. These procedures are generally time-consuming, tedious, and costly. Furthermore, they produce polynucleotide probes of varying lengths.

A chemical method for the preparation of polynucleotide probes thus has many advantages. Initial attempts to chemically label polynucleotides involved the introduction of a label onto the terminal sugar of a polynucleotide. A fluorescent label was attached to the 3' terminal end of RNA (Reines and Cantor ((1974)), Nucleic Acids Res., 1:767-786, and Wangyi et al. ((1980)), Scientia Sinica 23:1296-1308) and to the 5' terminal end of DNA (Smith et al. ((1985)) Nucleic Acids Res., 13:2399-2412). A biotin label was also attached to the 5' terminus of oligodeoxynucleotides. See Marasugi and Wallace (1984) DNA, 3:269-277, Chollet and Kawashima (1985), Nucleic Acids Res., 13:1529-1541, and Kempe et al. (1985) Nucleic Acids Res., 13:45-47. These methods had a disadvantage in that only one label was introduced per molecule of nucleic acid, thereby reducing the sensitivity of their detection.

Studies have been carried out on the effect of substituents at the 4-position of cytosine. Invariably, the studies have shown that the substituents decrease the binding energy between the modified cytosine and guanine. The monomethylation of the 4-amino group significantly decreases the T_{M} of a 1:1 complex of poly 4-methylcytosine + poly I. See Preparation and Properties of Some Methylated Polycytidylic Acids by R.L.C. Brimacombe and C.B. Reese in J. Mol. Biol. (1966), 18:529-540. The equilibrium constant for the formation of G:C basepairs is about eightfold greater than for the formation of G:4-methyl-C base pairs. Calculations show that the replacement of C by 4-methyl C in a base-paired polynucleotide destabilizes the Watson-Crick double-helical structure by about 1.0-1.8 kcal/mole per methyl substituent. See Effects of Methylation on the Stability of Nucleic Acid Conformations: Studies at the Monomer Level by J.D. Engel and P.H. Von Hippel, Biochemistry (1974) 13, 20, 4143-4158. By showing the destabilizing effect of substituents on the amino group of cytosine these publications teach that the use of polynucleotide probes comprising a reporting moiety at the 4-position of cytosine is not favorable.

Nevertheless, several papers have been published involving the labeling of DNA or RNA at internal positions by attaching a fluorescent or enzymatic moiety to the 4-position of cytosine. One paper reported the transamination of the 4-amino group with propylene diamine followed by the attachment of nitrobenzofuran (NBF) to the terminal amino group. The authors showed that polymers of poly (C, NBF-C) interacted with ribosomes by means of which the fluorescence of the NBF was enhanced. They also showed that the polymer can bind to poly I (inosine). However, the results showed that only 0.8 moles of I were bound per mole of C, NBF-C polymer, and then with a lower stability as compared to its binding to poly C. In addition, no data was shown indicating that the NBF-C pairs to G in random polymers. Furthermore the authors used bisulfite to carry out the transamination. Most important, the polynucleotide was not used as a probe for a particular polynucleotide. See "A Method for Linking Fluorescent Labels to Polynucleotides: Application to Studies of Ribosome-Ribonucleic Acid interactions", by D.E. Draper and L. Gold, Biochemistry (1980), 19:1774-1781. More recently, the authors also reported a transamination with ethylene diamine and bisulfite. See "Attachment of Reporter Groups to Specific, Selected Cytidine residues in RNA using a Bisulfite-Catalyzed Transamination Reaction", D.E. Draper (1984) Nucleic Acids Res., 12:989-1002.

Another paper described the attachment of a fluorescent moiety to a tRNA. The purpose was to study the effect of modifications of bases on the amino acid acceptor activity of tRNA's and to determine which cytosine bases, if any, are involved in recognition by the synthetase enzyme. The method involved the displacement of the 4-amino group of cytosine with carbonyl hydrazide followed by attachment of fluorescein to the hydrazide. The report did not involve a study on the effect of the attached fluorescent moiety on the base-pairing ability of the cytosine to guanine or on the use of such a polynucleotide as a probe. In addition, bridging moieties of potential diagnostic importance, such as biotin were not added. Furthermore, bisulfite, a mutagenic agent, was used to carry out the transamination. See "A New Method For Attachment of Fluorescent Probes to tRNA" by S.A. Reines and L.H. Schulman, Methods in Enzymology, vol. LIX, pp.146-156.

The attachment of enzymatic moieties, such as horseradish peroxidase and alkaline phosphatase, to a polynucleotide via a polyamine linker arm has also been published. The method did not involve a transamination or displacement step. Rather the polynucleotide was reacted with glutaraldehyde to form imine bonds. A drawback of this method is that the formation of the imine bond results in the loss of the amino hydrogen required for bonding to a complementary base. See Renz and Kurz (1984) Nucleic Acids Res., 12:3435-3444.

A method describing the photochemical addition of a biotin derivative to RNA or DNA via reaction with the amino groups of the bases has been published. Although relatively small amounts of biotin can be introduced into nucleic acids by this procedure, the introduction of higher levels of biotin among the various bases are likely to interfere with the hybridization of the probe to the target DNA. This is because the amino groups which react with biotin are the very same ones required for hydrogen bonding between base pairs. See Forster et al. (1985), Nucleic Acids Res., 13:745-761. Another method describes the attachment of N-acetoxy-N-2-acetylaminofluorene to the 8-position of guanine. Since N-acetoxy-N-2- acetylaminofluorene is a carcinogen, a probe comprising this compound is hazardous to prepare and use besides having safety and disposal problems. See Landegent et al. (1984), Exp. Cell Res., 153:61-72 and Tchen et al. (1984), Proc. Natl. Acad. Sci. USA, 81:3466-3470.

The conversion of cytosine bases of yeast tRNA into 4-deamino-4-acetohydrazidopyridinium cytosine bases has been reported. The purpose was to study the effect of such a modification the aminoacylation of tRNA for tyrosine, alanine, and valine. See "Modifications of Nucleosides and Nucleotides VI. The reaction of Girard-P Reagent with transfer RNA" by K. Kikugawa et. al., Biochim Biophys. Acta, (1967) 134:232-242.

The crosslinking of DNA with haloalkylhydrazides has been carried out. The crosslinking was between cytosine and guanosine. The object was to develop a general method for blocking specific genetic sequences to study in vitro the mechanisms of replication, transcription, and repair. No reporter molecule was attached to the hydrazide. See "Sequence-specific Crosslinking Agents for Nucleic Acids, use of 6-Bromo-5,5-dimethoxyhexanohydrazide for Crosslinking Cytidine to Guanosine and Crosslinking RNA to Complementary Sequences of DNA", by J. Summerton and P.A. Bartlett, J. Mol. Biol. (1978) 122:145-162.

The reaction of glycine hydrazide with DNA has been described. A relatively weak fluorescent group (dansyl) was introduced onto this hydrazide linker arm. However, this hydrazide was observed to be relatively non specific, since it reacted with deoxyadenosine, deoxycytidine and deoxyguanosine. Furthermore, the reaction was carried out at pH 4 and 37^{o}C for 42 hours, a period of time during which DNA con become partially depurinated leading to loss of specificity of the probe. In addition, the purpose of the fluorescent group was to permit the study of the interaction of DNA with proteins and other molecules and ions, and not for use as a probe. See Tsai and Marfey (1974), Physiol -Chem. Physics 6:191-204.

One recent report discloses a reaction of a polynucleotide moiety with a solution containing sodium bisulfite and ethylenediamine. The bisulfite adds reversibly to the 6-position of cytosine bases allowing the ethylenediamine to replace the 4-amino group by transamination. A disadvantage of the above procedure is that it uses sodium bisulfite. This compound is a known mutagen. In addition, it can convert the 4-amino groups of cytosine to oxo groups under certain conditions, causing to the polynucleotide to lose some of its base specificity. See "Novel Chemical Method For The Preparation of Nucleic Acids For Nonisotopic Hybridization" by R.P. Viscidi, C.J. Connelly, and R.H. Yolken in J. Clin. Microb. (1986) 23, pp 311-317.

Chemical Abstracts, 95:310 (1981), abstract no. 57576h discloses the use of a transamination reaction with bifunctional amines in the presence of bisulfite to attach side chains of variable length to the N⁴-position of single-stranded cytidine residues in Escherichia coli tRNA^{fmet}. EP-A 151 001 discloses the use of bisulfite to produce nonradiometric polynucleotide probes.

A method for preparing a probe comprising a reporting moiety attached to the 4-position of cytosine is briefly alluded to in European Patent Publication No. 0,097,373, published in January 4, 1984 by Dean Engelhardt et al. However, no specific details are given other than that the 4-amino group of cytosine can be alkylated by a reporting moiety.

Although considerable precedent exists for the chemical labeling of nucleotides, these studies have not resulted in the preparation of commercially significant DNA probes.

A synthetic method is disclosed herein for the preparation of a polynucleotide comprising a hydrazide compound. The hydrazide is attached to the 4-position of a former cytosine moiety to form a cytosine analog by displacing the 4-amino group. This method permits the synthesis of a polynucleotide probe, perferably, a polydeoxynucleotide probe, by attaching a reporting moiety to the hydrazide compound by means of a second functional group present in the hydrazide compound in which instance the hydrazide compound serves as a linker arm for attaching the reporting moiety to the polynucleotide.

The hydrazide compound can be attached to the 4-position of a cytosine base of a polynucleotide or to the 4-position of a cytidine triphosphate which is then incorporated into a nascent polynucleotide. In addition, a reporting moiety or bridging moiety can be joined to a hydrazide linker arm that is attached to the 4-position of a cytidine triphosphate and the modified cytidine triphosphate can then be incorporated into a nascent polynucleotide chain.

The polynucleotide probe can be prepared by either (a) reacting the hydrazide compound with a polynucleotide and attaching a reporting moiety to the hydrazide compound, or (b) attaching the reporting moiety to the hydrazide compound (or converting a functionality in the reporting moiety to a hydrazide functionality) and reacting the hydrazide compound with a polynucleotide.

It is an object of this invention to provide a method for the preparation of a polynucleotide probe, preferably, a polydeoxynucleotide probe, by reacting a hydrazide compound with a polynucleotide, preferably a polydeoxynucleotide, to displace an amino group of at least one cytosine base from said polynucleotide and attaching a reporting moiety to said hydrazide compound.

It is also an object of this invention to provide a method for the preparation of a polynucleotide probe, preferably, a polydeoxynucleotide probe, by attaching a reporting moiety to a hydrazide compound and reacting the modified hydrazide compound with a polynucleotide, preferably, a polydeoxynucleotide, to displace an amino group of at least one cytosine base.

It is an additional object of this invention to provide a method for the preparation of a polynucleotide probe be reacting a hydrazide compound with a polynucleotide at a pH from about 3.0 to about 8.0 at a reaction time from about 2-15 hours to displace an amino group from at least one cytosine base, and then attaching a reporting moiety to said hydrazide compound.

It is another object of this invention to provide a method for the preparation of a polynucleotide probe by reacting a hydrazide compound with a polynucleotide at a pH from about 3.0 to about 8.0 to displace an amino group from more than one cytosine base and attaching a reporting moiety to said hydrazide compound.

It is a further object of this invention to provide a method for the preparation a polynucleotide probe by reacting a dihydrazide compound with a polynucleotide to displace an amino group of at least one cytosine base and attaching a reporting moiety to said dihydrazide compound.

It is also an object of this invention to provide a method for the preparation of a polynucleotide probe by reacting a hydrazide compound with a polynucleotide at a pH from about 3.0 to about 8.0 to displace an amino group from at least one cytosine base, and attaching a bridging moiety to said hydrazide compound.

It is an additional object of this invention to provide a method for the preparation of a polynucleotide probe, preferably, a polydeoxynucleotide probe, by attaching a bridging moiety to a hydrazide compound and reacting said attached hydrazide compound with a polynucleotide, preferably, a polydeoxynucleotide, to displace an amino group of at least one cytosine base.

It is another object of this invention to provide a method for the preparation of a polynucleotide probe by reacting a hydrazide compound with a polynucleotide at a pH from about 3.0 to about 8.0 to displace an amino group from more than one cytosine base and attaching a bridging moiety to said hydrazide compound.

It is also an object of this invention to prepare polynucleotide probes, preferably, deoxypolynucleotide probes, by reacting a hydrazide compound with a polynucleotide, preferably a deoxypolynucleotide, to displace an amino group of at least one cytosine base from said polynucleotide and attaching a reporting moiety to said hydrazide compound.

It is a further object of this invention to prepare a polynucloetide probe by reacting a cytidine mono- or triphosphate with a hydrazide compound to displace the 4-amino group of the cytidine, incorporating the modified nucleotide triphosphate into a nascent polynucleotide chain, and attaching a reporting moiety to said hydrazide compound.

This invention relates to a method for the preparation of an entity comprising a polynucleotide and a hydrazide compound. The entity is prepared by displacing the 4-amino group of a cytosine moiety with the hydrazide functionality in the absence of bisulfite. This invention also relates to a method for chemically preparing a polynucleotide probe. The polynucleotide probe generally comprises a polynucleotide moiety and a reporting moiety which is attached to the polynucleotide moiety. The polynucleotide moiety of the probe has the ability to base-pair, i.e., hybridize to a complementary polynucleotide sequence of interest or target polynucleotide. The reporting moiety of the probe has or produces the means by which the presence of the target polynucleotide can be verified. The means can be, for example, fluorescence, phosphorescence, radioactivity, chromogen, electron density, or chemiluminescence.

The polynucleotide probe can be prepared in several ways. One way, which is preferred, is to react a hydrazide compound in the absence of bisulfite with a polynucleotide to displace a 4-amino group of at least one cytosine base. A reporting moiety is then directly attached to the hydrazide compound by means of a functional group other than the hydrazide present in the hydrazide compound. Alternatively, a reporting moiety is first attached to the hydrazide compound, and the hydrazide compound reacted with a polynucleotide to displace an amino group of at least one cytosine base. Other variations comprise attaching a reporting moiety indirectly to the hydrazide compound by means of a bridging moiety. The hydrazide compound serves as a linker arm for linking the polynucleotide to the reporting moiety.

### A. DESCRIPTION OF THE POLYNUCLEOTIDE

The polynucleotide containing the desired base sequence can be prepared enzymatically or synthetically. Enzymatic preparation involves the use of an appropriate template, a polymerase enzyme ans a mixture of the triphosphate precursors including one whose base portion is modified. One suitable method uses the M13 vector. These methods are well known to one skilled in the art and will not be discussed here. Synthetic methods involve attaching a linker arm to a predetermined unmodified polynucleotide. Such methods can provide a simple and easy method for large scale production of DNA probes. Inexpensive, readily available chemicals can be employed and theoretically, no special equipment is needed. The number of linker arms attached to the polynucleotide can be controlled by varying the amounts of each reactant and the reaction conditions.

Synhthetic methods also have the unique advantage in that the synthesis of the polynucleotide can be automated by instrumentation. It is conceivable that automation will permit the incorporation of a chemically synthesized nucleotide precursor containing a linker arm directly into a specific base sequence. However, the polynucleotide need not be prepared synthetically. It can be prepared enzymatically with polymerase enzyme by methods well known to those skilled in the art.

Manufacturers of polynucleotide synthesizing machines include Applied BioSystems, 850 Lincoln Centre Drive, Foster City, California 94404; Biosearch, Inc. 2980 Kerner Blvd., San Rafael, California 94901, and Beckman Instruments, 1050 Page Mill Road, Palo Alto, California 94304.

The most widely used chemical synthesis involves the phosphoramidite method because of its inherently high coupling efficiency and the stability of the starting materials. The starting material is the solid support derivatized with a 3'-hydroxyl terminal nucleoside. The nucleoside is attached to the silica to the 3'-OH. The 5'-hydroxyl is blocked with a dimethoxytrityl group. The synthesis is performed with the growing polynucleotide chain attached to a solid support so that excess reagents which are in the liquid phase can be removed by filtration, eliminating the need for purification steps between base additions.

The first steps of the synthesis cycle comprise:treating of the derivatized solid support with acid to remove the trityl group and generate a free 5'-OH; activating a predetermined nucleoside by adding its phosphoramidite derivative and a weak acid, tetrazole, to the reaction chamber, and reacting the freed 5'-OH with the activated nucleoside. The addition reaction is generally complete in less than two minutes at room temperature.

The next steps comprise: capping all sequences which did not undergo addition, and converting all phosphite internucleotide linkages to the more stable phosphates. This reaction is 100% complete in less than 30 seconds. After the oxidation step, the dimethoxytrityl group is removed and the cycle is repeated until the chain elongation is complete. At this point, the oligonucleotide is still bound to the solid support and has protecting groups on the phosphates and on the exocyclic amino groups of the bases A, G, and C. To produce biologically active DNA, the methyl groups on the phosphates are removed and the chains are cleaved from the support. After the solution containing the DNA is removed from the instrument, the protecting groups on the exocyclic amines of the bases are cleaved.

The degree of subsequent purification required depends on the application. With the 98-100% stepwise yields routinely obtained with the synthesizer, it is often not necessary to purify probe oligonucleotide containing from about 15 to about 25 bases. Other applications using longer oligonucleotides may require purification by electrophoresis or high pressure liquid chromatography (HPLC).

Polynucleotide synthesis using the phosphoramidite method provides a number of advantages. Firstly, the diisopropyl phosphoramidites are stable for a prolonged period and are nonhygroscopic. Secondly, coupling reactions using phosphoramidites can be carried out in acetonitrile (rather than in pyridine) which favors the keto form, thus protecting the bases from side-chain addition.

The polynucleotide should gernerally comprise at least about 12 to 15 bases. This is to impart specificity to the base sequence and make it selective as a probe. However, according to one manufacturer (Applied Biosystems), one can prepare polynucleotides up to about 200 bases.

### B. DESCRIPTION OF THE HYDRAZIDE COMPOUND

The hydrazide compound is attached to a polynucleotide by displacing the 4-amino group of a cytosine moiety.

The hydrazide compound can serve as a linker arm for attaching a polynucleotide moiety. The hydrazide compound when serving as a linker arm can contain as little as 6 atoms or can contain over 1000 atoms, preferably from about 6 atoms to about 100 atoms. The linker arm can be attached to the cytosine bases of either single-stranded polynucleotides or double-stranded polynucleotides that have been partially denatured.

The linker arm comprises a first hydrazide functionality and a second functionality other than a primary amino group that is attached to the carbon alpha of the carbonyl of the hydrazide, provided that the second functionality does not substantially react with the first hydrazide functionality of either the same or another molecule. The functionality can be a nucleophile, an electrophile, or a leaving group. Examples of functionalities include but are not limited to carboxylic esters, carboxylic acids thioesters, imides, ketones, aldehydes, epoxides, halides, N-hydroxysuccinimide esters, isocyanates, isothiocyanates, imidates, anhydrides, hydrazines, alkoxides, alkylamines, mercaptans, carboxylates, sulfonates, hydrazides, haloketones, diazonium salts, acylazides, nitrophenyl esters, sulfonyl chlorides and maleic imides. Preferred second functionalities include hydrazines, amines, alkylamines, mercaptans, carboxylates, and carboxylic esters. Particularly preferred second functionalities include hydrazines, amines, and alkylamines. The hydrazide compound can comprise, for example, a carbon-carbon double bond, carbon-nitrogen single bond, carbon-nitrogen double bond, carbon-carbon triple bond, carbon-oxygen single bond, carbon-sulfur single bond, or carbon-silicon single bond.

Preferred hydrazides compounds are those that are soluble in aqueous solutions. A particularly preferred hydrazide is carbonyl hydrazide. Other preferred hydrazides are amino acid hydrazides wherein the amino group is not α to the carbonyl group, and monohydrazides of dicarboxylic acids. It should be noted that oxalyl hydrazide is less preferred because of its poor solubility in aqueous media at the optimum pH required for reaction, while malonic and tartaric dihydrazides are less preferred because they decompose in aqueous solution.

### C. REACTION OF THE POLYNUCLEOTIDE WITH THE LINKER ARM

The linker arm for attaching a reporting moiety to a polynucleotide is a hydrazide compound. The 4-amino group of cytosine is displaced by the hydrazide compound in a displacement reaction. The conditions for the displacement reaction will vary with the particular hydrazide. Factors that affect the reaction are solubility of the hydrazide in aqueous solutions, reactivity of the hydrazide, and stability of the hydrazide under the reaction conditions. Hydrazides that are water soluble are preferred because the displacement reaction can be carried out in aqueous solutions. However, the reaction can also be carried out in a mixed solvent system. The pH range for the reaction can vary from about 3.0 to about 8.0, although from about 3.5 to about 6.0 is preferred. The temporature can vary from about 20^{o}C to about 90^{o}C, although from about 35^{o}C to about 50^{o}C is preferred. Generally, any buffer can also be present. However, the presence of bisulfite is not required - in fact, it is not desired, because it is a mutagen.

It is understood that displacement reactions carried out at low temperatures can require longer reaction times than those carried out at high temperatures. Similarly, reactions at acidic pH may proceed more rapidly than at neutral pH. However, any condition that permits displacement of the amino group by a hydrazide and that does not result in the alteration of the bases, cleavage of the glycosidic or phosphodiester bond, and decomposition of the hydrazide compound is suitable.

The stoichiometry of the reactants can vary. Generally, an excess of the hydrazide compound will be employed as compared to the number of cytosine bases. However, an expensive hydrazide compound may dictate the use of a stoichiometric ratio or even for an excess of base.

### D. ATTACHMENT OF THE REPORTING MOIETY TO THE LINKER ARM

The reporting moiety provides the means for detecting the presence of the target polynucleotide. The reporting moiety can be attached directly to the linker arm (hydrazide compound), or indirectly by means of one or more bridging moieties. The reporting moiety or bridging moiety can be attached to the linker arm or to a bridging moiety covalently or non-covalently. The reporting moiety can be attached to the linker arm following reaction of the linker arm with the polynucleotide or prior to reaction of the linker arm with the polynucleotide.

The reporting moiety can be attached directly and covalently to the linker arm by anyone of a number of functionalities present in the reporting moiety. Such functionalities include, but are not limited to, isothiocyanates, carbodiimides, sulfonyl chlorides, and n-hydroxysuccinimide esters. The functionality should be capable of reacting with the second functional group of the linker arm, i.e., the one other than the hydrazide functionality. An example is where the second functionality of the linker arm which is attached to a cytosine of a polynucleotide is an amino group, and the reporting moiety comprises an isothiocyanate functionality. The amino group reacts with the isothiocyanate to attach directly and covalently the linker arm to the reporting moiety. A number of reporting moieties are commercially available which already have the required functional group by means of which the reporting moiety can become attached to the linker arm. See, for example, the 1984 Catalog by Enzo Biochem Inc., 325 Hudson Street, New York, New York 10013.

The reporting moiety can also be attached to the linker arm indirectly and non-covalently. This can be, for example, where alkaline phosphatase, the reporting moiety, is covalently attached to an avidin molecule. Biotin, a bridging moiety, is covalently attached to a linker arm which is attached to a cytosine of a polynucleotide. The formation of a complex between biotin and avidin results in the reporting moiety becoming indirectly and non-covalently attached to the linker arm.

Examples of reporting moieties which are enzymes include, but are not limited to, hydrolases, esterases, phosphatases, peroxidases, catalases, glycosidases, oxidoreductases, proteases, lipases, and nucleases. Particularly prefered are the phosphatases, peroxidases, and oxidoreductases. Examples of fluorescent moieties include, but are not limited to, rhodamine B and fluorescein.

There are no limitations as to how many reporting moieties can be attached to the polynucleotide. The greater the number of reporting moieties, the greater is the sensitivity of the polynucleotide probe. However, in order to minimize steric hindrance, it is believed that there not be more than one reporting moiety per four sequential nucleotides.

### E. A METHOD FOR DETECTING TARGET POLYNUCLEOTIDES

Polynucleotide hybridization is based on complementary base-pairing. When single-stranded target polynucleotides are mixed either in solution or on a support with single-stranded polynucleotide probes, complementary base sequences pair to form double-stranded hybrid molecules. Detection of target polynucleotides by their hybridization to polynucleotide probes can be carried out, for example, by isolating the double-stranded polynucleotides from a sample and fixing them onto a support, for example, nitrocellulose, glass slide, plastic, paper or other synthetic polymer.

The fixed polynucleotides are mixed with a solution containing the polynucleotide probe, and the support is heated to about 80-90^{o}C to denature the polynucleotide double-strands. (The double-strands can also be denatured by means of alkali). The system which now contains the denatured target polynucleotide and the polynucleotide probe is allowed to cool to an appropriate temperature, to allow hybridization to take place. After sufficient time has elapsed for hybridization to be complete, which can be for ten minutes to several hours, the fixed target polynucleotide is washed to remove all unbound polynucleotide probes. The reporting moiety of the polynucleotide probe is now detected, either directly, for example, by means of radioactivity or fluorescence, or indirectly, for example, by means of a chromogen formed through an enzymatic reaction. See M. Grunstein and J. Wallas, METHODS IN ENZYMOLOGY, volume 68, R.Wu (Ed) (1979) pp. 375-469; A.R. Dunn, and J. Sambrook, METHODS IN ENZYMOLOGY, volume 65; part 1, (1980) pp. 468-478; Modified Nucleotides And Methods Of Preparing and Using The Same by D.C. Ward, A.A. Waldrop, and P.R. Langer, European Patent Publication Number 0,063,879, published November 3, 1982; DNA Probes for Infectious Disease by A.J. Berry and J.B. Peter, Diagnostic Medicine (March, 1984) pp. 1-8; and Recombinant DNA Technology:Some Applications In Clinical Microbiology by Wie-Shing Lee and James L. Bennington, Laboratory Management (April, 1985) pp. 21-26, which are hereby incorporated by reference.

The priority document for EP 0,063,879 is U.S. Application Serial No. 255223, filed on April 17, 1981, which was abandoned in favor of continuation application serial No. 496,915, filed on May 23,1983. Serial No. 496,915 issued as U.S. Patent No. 4,711,955.

A second way is to detect the target polynucleotide in situ. The tissue specimen comprising the target polynucleotide is fixed to a glass slide. The specimen can be fixed, for example, either in acetone or in CARNOY's B fixative (10% acetic acid, 30% chloroform, 60% methanol). The fixative is allowed to remain in contact with the specimen for about 5 minutes. Excess fixative is tapped off the slide and the slide is allowed to air dry.

A solution containing a polynucleotide probe with a fluorescent molecule as its reporting moiety is added to the specimen on the support and the specimen is covered with a coverslip. The covered slide is heated to about 90-95^{o}C for approximately 0.5-2 minutes to denature all the double-stranded polynucleotides. The slide is then removed from the heating block and hybridization is allowed to proceed at room temperature for about 10 minutes. The specimen is first washed with stringency solutions to remove all nonhybridized polynucleotide probes and then washed with buffer to remove the stringency solution. The slide is finally examined under a fluorescent microscope. The presence of fluorescence in the specimen indicates the presence of the target polynucleotide.

Alternatively, the reporting moiety can be an enzyme. The enzyme cannot be present during the heating step because it would become denatured by the heat. The enzyme can be added however following the stringency wash. An example of such a method is where biotin, a bridging moiety, is attached to a linker arm which is attached to cytosine, and the enzyme reporting moiety is attached to avidin. The polynucleotide probe comprising the biotin is allowed to mix with the specimen is added to the fixed specimen following the stringency wash. The presence of the target polynucleotide will result in the formation of a polynucleotide hybrid wherein one of the polynucleotides comprises biotin. The addition of the avidin to which the enzyme is bound to the specimen results in the avidin complexing to the biotin. The addition of a substrate to the enzyme and the appearance of a color verifies the presence of the target polynucleotide.

### EXAMPLES:

The following examples are by way of illustration and not by way of limitation.

### Chemical labeling of single-stranded M13 DNA

### Reagents

Carbohydrazide (purchased from Aldrich Chemical Comp.) solution was freshly prepared by dissolving 0.36 g of carbohydrazide in 1.85 ml of distilled water and slowly adding 0.15 ml of concentrated HCl. The final concentration of carbohydrazide was 2M and the final pH of the solution was between 4.15 and 4.25.

### Reaction of DNA with carbohydrazide

To 300 ug of single-stranded M13 DNA in 50 ul of distilled water was added 600 ul of carbohydrazide solution. The mixture was vortexed and then incubated at 37^{o}C for 6 hours. The product was neutralized with 30 ul of 10N NaOH and the DNA was precipitated by addition of 1.4 ml of cold alcohol. The DNA was redissolved in 0.5 ml of 1M NH₄ Ac and then precipitated by addition of 1.0 ml of cold alcohol. This procedure was repeated once. This carbohydrazide-modified-DNA was analyzed by gel electrophoresis. The appearance of a single UV reactive band indicated that the majority of the DNA was not degraded.

### Preparation of biotin labelled single-stranded M13 DNA

To 75 ug of carbohydrazide modified DNA (CH DNA) in a mixture of 100 ul of 90 mM borate buffer (pH 8.5) containing 10 mM EDTA and 30 ul of DMSO was added 30 ul of biotin solution (40mg/ml in DMSO). The mixture was kept at room temperature overnight. The modified DNA was then precipitated by the addition of 320 ul of cold alcohol. After 20 hours at -20°C, the DNA was collected by centrifugation and then washed once with 300 ul of cold alcohol (70%). After lyophilization, the biotinylated DNA probe was stored in 150 ul of tris EDTA buffer (10 mM tris HCl (pH 7.5) mM EDTA).

### Preparation of fluorescein labelled single-stranded M13 DNA

125 ug of carbohydrazide modified M13 DNA was dissolved in 200 ul of borate buffer, and 100 ug of fluorescein isothiocyanate (FITC) solution (10mg/ml DMF) was added. The reaction mixture was incubated at room temperature for 20 hours. The reaction mixture was purified by passage through a G-50 column (10 cm × 1 cm), using 50 mM triethylammonium bicarbonate (pH 7.5) as eluting buffer. The fluorescein labelled DNA was obtained by lyophilization of the solvent. The method for the measurement of the number of fluorescein molecules on the DNA was adapted from that of Reines, S.A. and Schulman, L.H., Methods Enzymol. (1959) 59:146. This measurement indicated that one fluorescein molecule was incorporated per 10 to 15 bases of DNA.

### Preparation of Texas Red labelled single-stranded M13 DNA

The labeling procedure was adapted from that of Titus, J.A., Hangland, R., Sharrow, S.O., and Sega., D.M., J. Immunol. Methods (1982) 50:193. One hundred twenty five ug of carbohydrazide-modified-M13 DNA was dissoved in 250 ul of borate buffer (90 mM, pH 8.5) containing 10 mM of ethylenediaminetetraacetic acid (Na⁺, pH 7.5.). Ten mg of Texas Red-celite mixture (w/w, 1:9) was suspended in the DNA solution. The reaction mixture was stirred at 4°C in the dark overnight (about 16 hours). Texas-Red labeled DNA was purified by elution with 10 mM of triethylammonium bicarbonate, pH 7.5, through a G-50 column (0.9 × 10 cm). The eluant was removed by repeated lyophilization. The DNA thus obtained was assayed by measurement of the ratio of the absorption at 590 nm to 260 nm. This measurement indicated that one fluorophore was incorporated per 10 to 15 bases of DNA.

### Microtiter-plate Hybridization and Detection Using Single-Stranded Biotinylated DNA as Probe

This method was adapted from that disclosed in patent application serial number 732,374 which is a CIP of patent application serial number 461,469. Target double-stranded RF M13 DNA was heat denatured (boiled for 5 minutes and chilled quickly in ice-water) and then immobilized on a microtiter plate in 1M ammonium acetate. The ammonium acetate buffer was decanted off and the plate was washed twice with 2 X SSC (room temperature). The biotinylated M13 probe (0.25 ug/ml), prepared as described above, was added and hybridization was carried out at 37°C for 16 hours in hybridization buffer (30% deionized formamide in 4 X SSPE, 5% dextran sulfate, 1% triton X-100 and 1mg/ml of sonicated, heat denatured salmon sperm DNA). The plate was washed twice with 2 X SSC, 0.1% triton X-100 at 65°C. The plate was incubated at 37°C for 30 minutes with blocking solution (20ug/well, PBS (Phosphate Buffered Saline), 2% Bovine Serum Albumin, 0.1% of Triton X-100 and 5mM EDTA). After the blocking solution was removed, the plate was incubated at 37°C for 30 minutes with horseradish peroxidase-streptavidin complex (diluted 1:5,000 in PBS, 1% Bovine Serum Albumin, 0.1% Triton X-100 and 5mM EDTA; 50ug/well). A solution of o-phenylenediamine which was used as substrate for horseradish peroxidase was prepared by dissolving 4mg of o-phenylenediamine and 25 ul of H₂O₂ (10%) in 20 ml of phosphate-citrate buffer (pH 6). The plate was incubated with this o-phenylenediamine solution for 5-10 minutes at room temperature and the reaction was stopped by adding 4N sulfuric acid solution. The detection of the target DNA was measured by reading the visible light absorbance at 490nm.

The sensitivity of the biotinylated M13 DNA probes was compared with that of biotin nick-translated M13 probe, prepared by the procedure of Langer, P.R., Waldrop, A.A. and Ward, D.C. (1981) Proc. Natl. Acad. Sci. USA 78:6633. The chemically biotinylated M13 DNA probe (prepared as described above) was at least two times more sensitive than the nick-translated M13 DNA probe (with 24% of the thymidine nucleotides substituted with Bio-11-dUTP) in the detection of target M13 RF DNA. The fact that a sensitive, specific probe could be produced by this labeling method indicates that hydrogen bonding of the modified cytosine bases to the complementary guanine residues must be taking place.

### Carbohydrazide Modification of Double-Stranded M13 RF DNA M13 RF DNA (24µg/80ul in tris-HCl (10 mM pH 7.5)-EDTA (1 mM) was heat denatured by boiling it at 100^{o}C for 5 minutes and then cooling it to 0^{o} in ice water. The DNA was then dissolved in 400 ul of 2M carbohydrazide solution. The mixture was incubated at 37^{o}C for 6 hours and the excess reagent was removed by repeated alcohol precipitation as described above. Twenty ug of carbohydrazide modified DNA were obtained (83%).

### Biotinylation of carbohydrazide-modified double-stranded M13 (RF) DNA

Carbohydrazide-modified double-stranded M13 (RF) DNA (20ug in 50ul of distilled water) was heat denatured and then added to 0.15 ml of a solution containing borate buffer and DMSO (2:1). To the above solution was added 50 ul of biotin-aminocaproic acid N-hydroxy succinimide ester (ENZOTIN®) solution (40 mg/ml in DMSO). The mixture was incubated for 15 hours at 37^{o}C and the biotinylated M13 RF DNA was isolated by the standard alcohol precipitation procedure. Ten ug of biotinylated DNA were obtained (50% yield).

### Dot-Blot Hybridization and Detection Using Double-Stranded M13 DNA Probe

The method was modified from that of Thomas, P.S., (1983) Methods Enzymol. 100:255-266. Nitrocellulose filters were soaked in water followed by 20 X SSC (SSC:0.15M NaCl; 0.015M sodium citrate). Dry filters were spotted with denatured M13 (RF) DNA (boiled for 10 minutes and then chilled quickly in ice-water) in 0.1 mM EDTA (1 ul), dried, and baked at 80^{o}C in vacuo for 2 hours. Filters were then pre-hybridized at 65^{o}C for at least 2 hours in pre-hybridization buffer (5 X SSC, 0.1% SDS, 1.0 mg/ml each of bovine serum albumin, ficoll 400 and polyvinylpyrrolidone, MW, 40,000, and 1 mg/ml of sonicated, heat denatured [boiled for 10 minutes and quickly chilled in ice-water] salmon sperm DNA). Hybridization was carried out in a shaken water bath at 65°C for 20 hours in the pre-hybridization buffer containing 50 ng/ml of heat denatured biotin-labelled M13 RF probe. Filters were washed while shaken in 2 X SSC, 0.1% SDS (2 x 15 minutes, at 65°C), and then washed twice at room temperature with 2 X SSC solutions.

The colorimetric detection method was adapted from that of Leary, J.J., Brigati, D.J. and Ward, D.C. (1983) Proc. Natl. Acad. Sci. 80:4045. The filters were incubated at 37°C for 30 minutes with horseradish peroxidase-streptavidin complex in the buffer containing phosphate buffered saline (0.13 M of sodium chloride, 0.007 M dibasic sodium phosphate and 0.003 M monobasic sodium phosphate and 1% of bovine serum albumin). After washing the filters three times with high salt buffer (0.5 M sodium chloride, 10mM phosphate buffer [pH 6.5], 0.1% bovine serum albumin, 0.005% Tween-20), the filters were developed with diaminobenzidine tetrahydrochloride in 10 mM Tris buffer (pH 7.5). The reaction was terminated by washing the filters with tap water and blotting them dry. The sensitivity of detection of target double-stranded M13 RF DNA was 400 pg. Under the same conditions, the sensitivity of detection using a biotin nick-translated M13 probe was 80 pg of target M13 RF DNA.

### Reaction of Cytidine Monophosphate (CMP) With Carbohydrazide

Carbohydrazide solution was prepared by dissolving 9.0 gm of carhohydrazide in 50ml of water and adjusting the pH to pH 4.2 with concentrated HCl (about 5.0ml). Cytidine monophosphate (1.5g, 4.1 mmoles) was dissolved in 40 ml of 2M carbohydrazide solution and the mixture was incubated at 37°C for 20 hours. Paper chomatography analysis (3M Whatman cellulose paper, elution with 0.1 N NaAC [pH 6.0]/CH₃CN,40:60) indicated that all the CMP (RF=0.32) was consumed and a new compound was generated with an RFof 0.25. The reaction mixture was diluted to 400 ml with water and then treated with 10ml of 0.5N lanthanum nitrate. The mixture was kept in a cold room for 3 hours and the white precipitate was collected by centrifugation. The supernatant was decanted off, and the white precipitate was suspended in 250 ml of water and 25 g of Dowex 50- X 8 (Na+) was added. The mixture was stirred continuously until a clear solution resulted. By paper chromatography, as described earlier, this solution was shown to contain a single compound which was positive to the hydrazide test (spraying with reagent containing 1 mg picryl sulfonic acid in 1 ml of 0.1 M Na borate buffer generated an orange red color. The amount of product obtained was 15,600 OD₂₇₀ units (1.56 nmoles), 38% based on assumed e270 = 10,000 at pH 7.5). The cabohydrazide modified CMP was obtained as white powder after lyophilization. A sample was further purified by passage through an anion exchange column (DE52 column, eluted with a linear gradient from 0.01 M to 0.3 M triethyl-ammonium bicarbonate, pH 7.5). The desired product was eluted from the column at about 0.08 M triethylammonium bicarbonate and lyophilized to give a white powder.

HPLC analysis (SAX column, 5% methanol in 0.05M potassium phosphate pH 3.5) gave a single peak, retention time= 13.5 min,) (retention time of starting material = 9.1 min). TLC on cellulose paper (0.1N NH₄AC, pH 5/acetonitrile, 40:60) RF=0.29 (starting material RF=0.32). The presence of biotin in the product was detected by spraying the cellulose paper with a solution of p-dimethylaminocinnamaldehyde (0.2%) in ethanol containing 6% conc. sulfuric acid. Biotin containing compounds give a pink spot with this reagent. NMR (in D₂0): δ 8.7 (NH,S); δ 8.0 (C₆H,d); δ 7.8 (NH,S); δ 5.95 (C₅H,d); δ 5.90 (C₁'H,m); δ 3.8-4.0 (C₂'H,C₃'H,C₅'H,m).

### Biotinylation of Carbohydrazide Modified CMP (CH-CMP)

10 umoles of CH-CMP was dissolved in 0.4ml of 0.1 N sodium borate (pH 9.0) and 4 mg of biotin -ε -amino-carproic acid NHS ester (Enzotin®, Enzo Biochem) in 30 ul of DMSO was added. The mixture was stirred at 4^{o}C overnight and an additional 6 mg of biotin -ε -amino-caproic acid NHS ester in 50 ul DMSO was added. After the reaction mixture was purified by DE 52 anion exchange cellulose column chromatography using a linear gradient of triethylammonium bicarbonate buffer, pH 7.5, 0.01 - 0.15M, the fractions which were eluted at 0.06M buffer concentration were combined and evaporated to dryness to give the product as a white foam.

## Claims

1. A method for the modification of a nucleotide, oligonucleotide, or polynucleotide having at least one cytosine bass therein comprising the step of contacting said nucleotide, oligonucleotide, or polynucleotide with a hydrazide compound comprising a first functionality which is a hydrazide and any second functionality under conditions which permit the displacement of the 4-amino group of at least one of said cytosine bases with said hydrazide functionality to form a hydrazide-modified nucleotide, oligonucleotide or polynucleotide, with the proviso that said contacting step is carried out in the absence of bisulfite, and with the further proviso that when said second functionality is a primary amino group and when said hydrazide compound contains a carbon alpha to said hydrazide functionality, said primary amino group is not attached to said alpha carbon.

2. The method of Claim 1 wherein a first bridging moiety is attached to said hydrazide compound prior to said contacting step.

3. The method of Claim 2 wherein said first bridging moiety is biotin.

4. The method of Claim 2 wherein a second bridging moiety is attached to said first bridging moiety.

5. The method of Claim 4 wherein said second bridging moiety is attached non-covalently.

6. The method of Claim 5 wherein said first bridging moiety is biotin and wherein said second bridging moiety is avidin or streptavidin.

7. The method of Claim 1 wherein a reporting moiety is attached to said hydrazide compound prior to said contacting step.

8. The method of Claim 1 which further comprises attaching a reporting moiety to said hydrazide-modified nucleotide, oligonucleotide, or polynucleotide.

9. The method of any one of Claims 1, 2 or 8 which further comprises incorporating said hydrazide-modified nucleotide, oligonucleotide, or polynucleotide into a nucleotide sequence.

10. The method of any one of Claims 1, 7 or 8 wherein said oligonucleotide, or polynucleotide has more than one cytosine base.

11. The method of Claim 2 further comprising the step of attaching a reporting moiety to said bridging moiety.

12. The method of Claim 1 wherein said polynucleotide is hybridized to a complementary polynucleotide.

13. The method of Claim 1 wherein said hydrazide compound contains from about 6 atoms to about 1000 atoms, preferably from about 6 atoms to about 100 atoms.

14. The method of Claim 1 wherein said hydrazide compound is carbonyl hydrazide.

15. The method of Claim 1 wherein said hydrazide compound is water soluble.

16. The method of Claim 1 wherein said second functionality is a carboxylic ester , carboxylic acid thioester , imide , ketone , aldehyde , epoxide , halide , N-hydroxysuccinimide ester , isocyanate , isothiocyanate , imidate , anhydride , hydrazine , alkoxide , amine , alkylamine , mercaptan , carboxylate , sulfonate , hydrazide , haloketone , diazonium salt , acylazide , nitrophenyl ester , sulfonyl chloride or maleic imide .

17. The method of Claim 16 wherein said second functionality is a hydrazine amine , alkylamine , mercaptan , carboxylate , or carboxylic ester .

18. The method of Claim 17 wherein said second functionality is a hydrazine, amine, or alkylamine.

19. The method of Claim 18 wherein said second functionality is a hydrazine.

20. The method of Claim 1 wherein said hydrazide compound comprises at least one carbon-carbon double bond , carbon-nitrogen single bond , carbon-nitrogen double bond , carbon-carbon triple bond , carbon-oxygen single bond , carbon-sulfur single bond , and/or carbon-silicon single bond.

21. The method of Claim 1 wherein said method is carried out in a solution having a pH from about 3.0 to about 8.0, preferably from about 3.5 to about 6.0.

22. The method of Claim 1 wherein said method is carried out in a solution having a temperature of from about 20^{o}C to about 90^{o}C, preferably from about 35°C to about 50°C.

## Patentansprüche

1. Verfahren zur Modifikation eines Nucleotids, Oligonucleotids oder Polynucleotids mit mindestens einer Cytosinbase darin, umfassend das Inkontaktbringen des Nucleotids, Oligonucleotids oder Polynucleotids mit einer Hydrazidverbindung, die eine erste Funktionalität, nämlich Hydrazid, und eine beliebige zweite Funktionalität aufweist, unter Bedingungen, die das Ersetzen der 4-Aminogruppe von mindestens einer der Cytosinbasen mit der Hydrazidfunktionalität erlaubt, so daß ein Hydrazidmodifiziertes Nucleotid, Oligonucleotid oder Polynucleotid erhalten wird, mit der Maßgabe, daß das Inkontaktbringen in Abwesenheit von Bisulfit durchgeführt wird, und mit der weiteren Maßgabe, daß, wenn die zweite Funktionalität eine primäre Aminogruppe ist und wenn die Hydrazidverbindung ein Kohlenstoffatom in α-Stellung zu der Hydrazidfunktionalität aufweist, die primäre Aminogruppe nicht an das α-Kohlenstoffatom gebunden ist.

2. Verfahren nach Anspruch 1, wobei eine erste Brückeneinheit vor dem Inkontaktbringen an die Hydrazidverbindung geknüpft wird.

3. Verfahren nach Anspruch 2, wobei die erste Brückeneinheit Biotin ist.

4. Verfahren nach Anspruch 2, wobei eine zweite Brückeneinheit an die erste Brückeneinheit geknüpft wird.

5. Verfahren nach Anspruch 4, wobei die zweite Brückeneinheit nicht kovalent verknüpft wird.

6. Verfahren nach Anspruch 5, wobei die erste Brückeneinheit Biotin ist und die zweite Brückeneinheit Avidin oder Streptavidin ist.

7. Verfahren nach Anspruch 1, wobei eine Nachweiseinheit vor dem Inkontaktbringen an die Hydrazidverbindung geknüpft wird.

8. Verfahren nach Anspruch 1, welches weiterhin das Anknüpfen einer Nachweiseinheit an das Hydrazid- modifizierte Nucleotid, Oligonucleotid oder Polynucleotid umfaßt.

9. Verfahren nach einem der Ansprüche 1, 2 oder 8, welches weiterhin den Einbau des Hydrazid-modifizierten Nucleotids, Oligonucleotids oder Polynucleotids in eine Nucleotidsequenz umfaßt.

10. Verfahren nach einem der Ansprüche 1, 7 oder 8, wobei das Oligonucleotid oder Polynucleotid mehr als eine Cytosinbase aufweist.

11. Verfahren nach Anspruch 2, welches weiterhin das Anknüpfen einer Nachweiseinheit an die Brückeneinheit umfaßt.

12. Verfahren nach Anspruch 1, wobei das Polynucleotid mit einem komplementären Polynucleotid hybridisiert wird.

13. Verfahren nach Anspruch 1, wobei die Hydrazidverbindung etwa 6 bis etwa 1000 Atome, vorzugsweise etwa 6 bis etwa 100 Atome, enthält.

14. Verfahren nach Anspruch 1, wobei die Hydrazidverbindung Carbonylhydrazid ist.

15. Verfahren nach Anspruch 1, wobei die Hydrazidverbindung wasserlöslich ist.

16. Verfahren nach Anspruch 1, wobei die zweite Funktionalität ein Carbonsäureester, Carbonsäurethioester, Imid, Keton, Aldehyd, Epoxid, Halogenid, N-Hydroxysuccinimidester, Isocyanat, Isothiocyanat, Imidat, Anhydrid, Hydrazin, Alkoxid, Amin, Alkylamin, Mercaptan, Carboxylat, Sulfonat, Hydrazid, Haloketon, Diazoniumsalz, Acylazid, Nitrophenylester, Sulfonylchlorid oder Maleinimid ist.

17. Verfahren nach Anspruch 16, wobei die zweite Funktionalität ein Hydrazinamin, Alkylamin, Mercaptan, Carboxylat oder Carbonsäureester ist.

18. Verfahren nach Anspruch 17, wobei die zweite Funktionalität ein Hydrazin, Amin oder Alkylamin ist.

19. Verfahren nach Anspruch 18, wobei die zweite Funktionalität ein Hydrazin ist.

20. Verfahren nach Anspruch 1, wobei die Hydrazidverbindung mindestens eine Kohlenstoff-Kohlenstoff-Doppelbindung, Kohlenstoff-Stickstoff-Einfachbindung, Kohlenstoff-Stickstoff-Doppelbindung, Kohlenstoff-Kohlenstoff-Dreifachbindung- Kohlenstoff-Sauerstoff-Einfachbindung, Kohlenstoff-Schwefel-Einfachbindung und/oder Kohlenstoff-Silicium-Einfachbindung umfaßt.

21. Verfahren nach Anspruch 1, wobei das Verfahren in einer Lösung mit einem pH-wert von etwa 3,0 bis etwa 8,0, vorzugsweise etwa 3,5 bis etwa 6,0, durchgeführt wird.

22. Verfahren nach Anspruch 1, wobei das Verfahren in einer Lösung mit einer Temperatur von etwa 20°C bis etwa 90°C, vorzugsweise etwa 35°C bis etwa 50°C, durchgeführt wird.

## Revendications

1. Procédé de modification d'un nucléotide, d'un oligonucléotide ou d'un polynucléotide comportant au moins une base cytosine, selon lequel on met en contact le nucléotide, l'oligonucléotide ou le polynucléotide, avec un composé dérivé d'hydrazide comprenant une première fonction hydrazide et une deuxième fonction quelconque, dans des conditions permettant le déplacement du groupe 4-amino d'au moins l'une des bases cytosine avec la fonction hydrazide, pour former un nucléotide, un oligonucléotide ou un polynucléotide modifié avec un hydrazide, à condition que l'opération de mise en contact soit effectuée en l'absence de bisulfite, et en outre à condition que lorsque la deuxième fonction est un groupe amino primaire et que le composé dérivé d'hydrazide contient un atome de carbone en alpha par rapport à la fonction hydrazide, le groupe amino primaire ne soit pas lié à l'atome de carbone alpha.

2. Procédé selon la revendication 1, dans lequel un premier fragment de pontage est lié au composé dérivé d'hydrazide avant l'opération de mise en contact.

3. Procédé selon la revendication 2, dans lequel le premier fragment de pontage, est la biotine.

4. Procédé selon la revendication 2, dans lequel un deuxième fragment de pontage est lié au premier fragment de pontage.

5. Procédé selon la revendication 4, dans lequel le deuxième fragment de pontage, est lié de manière non covalente.

6. Procédé selon la revendication 5, dans lequel le premier fragment de pontage, est la biotine, et dans lequel le deuxième fragment de pontage, est l'avidine ou la streptavidine.

7. Procédé selon la revendication 1, dans lequel un fragment indicateur est lié au composé dérivé d'hydrazide avant l'opération de mise en contact.

8. Procédé selon la revendication 1, comprenant en outre la liaison d'un fragment indicateur au nucléotide, à l'oligonucléotide ou au polynucléotide modifié avec un hydrazide.

9. Procédé selon la revendication 1,2 ou 8, comprenant en outre l'incorporation du nucléotide, de l'oligonucléotide ou du polynucléotide modifié avec un hydrazide dans une séquence nucléotidique.

10. Procédé selon la revendication 1, 7 ou 8, dans lequel l'oligonucléotide ou le polynucléotide comprend plus d'une base cytosine.

11. Procédé selon la revendication 2, comprenant en outre l'opération de liaison d'un fragment indicateur au fragment de pontage.

12. Procédé selon la revendication 1, dans lequel le polynucléotide est hybridé avec un polynucléotide complémentaire.

13. Procédé selon la revendication 1, dans lequel le composé dérivé d'hydrazide contient d'environ 6 atomes à environ 1000 atomes, de préférence d'environ 6 atomes à environ 100 atomes.

14. Procédé selon la revendication 1, dans lequel le composé dérivé d'hydrazide, est le carbonyl hydrazide.

15. Procédé selon la revendication 1, dans lequel le composé dérivé d'hydrazide, est soluble dans l'eau.

16. Procédé selon la revendication 1, dans lequel la deuxième fonction est un ester d'acide carboxylique, un thioester d'acide carboxylique, un imide, une cétone, un aldéhyde, un époxyde, un halogénure, un ester avec le N-hydroxysuccinimide, un isocyanate, un isothiocyanate, un imidate, un anhydride, une hydrazine, un alcoolate, une amine, une alkylamine, un mercaptan, un carboxylate, un sulfonate, un hydrazide, une halocétone, un sel de diazonium, un acylazide, un ester nitrophénylique, un chlorure de sulfonyle ou un imide maléique.

17. Procédé selon la revendication 16, dans lequel la deuxième fonction, est une hydrazine, une amine, une alkylamine, un mercaptan, un carboxylate ou un ester d'acide carboxylique.

18. Procédé selon la revendication 17, dans lequel la deuxième fonction, est une hydrazine, une amine ou une alkylamine.

19. Procédé selon la revendication 18, dans lequel la deuxième fonction, est une hydrazine.

20. Procédé selon la revendication 1, dans lequel le composé dérivé d'hydrazide, comprend au moins une double liaison carbone-carbone, une simple liaison carbone-azote, une double liaison carbone-azote, une triple liaison carbone-carbone, une simple liaison carbone-oxygène, une simple liaison carbone-soufre et/ou une simple liaison carbone-silicium.

21. Procédé selon la revendication 1, mis en oeuvre dans une solution ayant un pH d'environ 3,0 à environ 8,0, de préférence d'environ 3,5 à environ 6,0.

22. Procédé selon la revendication 1, mis en oeuvre dans une solution ayant une température d'environ 20 °C à environ 90 °C, de préférence d'environ 35 °C à environ 50 °C.
